(19) **Europäisches Patentamt European Patent Office Office européen des brevets**

(11) **EP 4 567 403 A1**

(12) **EUROPEAN PATENT APPLICATION**
published in accordance with Art. 153(4) EPC

(43) Date of publication:
**11.06.2025 Bulletin 2025/24**

(21) Application number: **23849994.1**

(22) Date of filing: **27.07.2023**

(51) International Patent Classification (IPC):
**G01N 17/00** (2006.01)   **G01N 33/24** (2006.01)

(52) Cooperative Patent Classification (CPC):
**G01N 17/00; G01N 33/24**

(86) International application number:
**PCT/JP2023/027501**

(87) International publication number:
**WO 2024/029439 (08.02.2024 Gazette 2024/06)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.08.2022 JP 2022125516**

(71) Applicant: **Kubota Corporation**
**Osaka 556-8601 (JP)**

(72) Inventors:
• **OCHI, Takatoshi**
  **Amagasaki-shi, Hyogo 660-0095 (JP)**
• **OKUMURA, Yuta**
  **Amagasaki-shi, Hyogo 660-0095 (JP)**

(74) Representative: **Bittner, Thomas L.**
**Boehmert & Boehmert**
**Anwaltspartnerschaft mbB**
**Pettenkoferstrasse 22**
**80336 München (DE)**

(54) **BURYING ENVIRONMENT CLASSIFICATION MAP CREATION DEVICE, BURYING ENVIRONMENT IDENTIFICATION DEVICE, BURYING ENVIRONMENT CLASSIFICATION MAP CREATION METHOD, BURYING ENVIRONMENT IDENTIFICATION METHOD AND PROGRAM**

(57)   A burial environment classification map creation apparatus (a second burial environment classification map creation unit (63)) includes a first soil identification unit (63a) that identifies a groundwater permeable soil which is a soil permeable to groundwater, and a second soil identification unit (63b) that identifies, from the groundwater permeable soil, a soil which is located within a predetermined distance from a salt-containing water source and has an elevation within a predetermined height as a highly corrosive soil which is highly corrosive to a pipe. (Fig. 2)

FIG.2

EP 4 567 403 A1

## Description

Technical field

The present disclosure relates to a burial environment classification map creation apparatus, a burial environment identification apparatus, a burial environment classification map creation method, a burial environment identification method, and a program.

Background

A pipe, such as a water pipe, is buried in the soil. During long-term use of the pipe, the pipe may be subject to corrosion. Japanese Patent Laying-Open No. 2007-107882 (PTL 1) discloses a method of predicting a degree of corrosion of a buried pipe.

Citation list

Patent literature

PTL 1: Japanese Patent Laying-Open No. 2007-107882

Summary of the invention

Technical problem

An object of the present disclosure is to provide a burial environment classification map creation apparatus, a burial environment identification apparatus, a burial environment classification map creation method, a burial environment identification method, and a program, which makes it possible to more accurately predict a degree of corrosion of a buried pipe.

Solution to the problem

A burial environment classification map creation apparatus according to an embodiment of the present disclosure includes: a first soil identification unit that identifies a groundwater permeable soil which is a soil permeable to groundwater; and a second soil identification unit that identifies, from the groundwater permeable soil, a soil which is located within a predetermined distance from a salt-containing water source and has an elevation within a predetermined height as a highly corrosive soil which is highly corrosive to a pipe.

A burial environment identification apparatus according to an embodiment of the present disclosure includes: a buried pipe data read unit that reads buried pipe data including a position of a buried pipe; and a burial environment identification unit that identifies a burial environment of the buried pipe by applying the buried pipe data to a burial environment classification map created by the burial environment classification map creation apparatus of the present disclosure.

A burial environment classification map creation method according to an embodiment of the present disclosure includes: a step of identifying a groundwater permeable soil which is a soil permeable to groundwater; and a step of identifying, from the groundwater permeable soil, a soil which is located within a predetermined distance from a salt-containing water source and has an elevation within a predetermined height as a highly corrosive soil which is highly corrosive to a pipe.

A burial environment identification method according to an embodiment of the present disclosure includes: a step of reading buried pipe data including a position of a buried pipe; and a step of identifying a burial environment of the buried pipe by applying the buried pipe data to a burial environment classification map created by the burial environment classification map creation method of the present disclosure.

A program according to an embodiment of the present disclosure causes a processor to execute each step of the burial environment classification map creation method of the present disclosure.

A program according to an embodiment of the present disclosure causes a processor to execute each step of the burial environment identification method of the present disclosure.

Advantageous effects of the invention

According to the burial environment classification map creation apparatus, the burial environment identification apparatus, the burial environment classification map creation method, the burial environment identification method, and the program of the present disclosure, it is possible to more accurately predict a degree of corrosion of a buried pipe.

Brief description of drawings

Fig. 1    is a schematic diagram illustrating a hardware configuration of a probability-of-water-leakage-accidents prediction apparatus according to an embodiment;

Fig. 2    is a block diagram illustrating a functional configuration of a probability-of-water-leakage-accidents prediction apparatus according to an embodiment;

Fig. 3    is a block diagram illustrating a functional configuration of a storage unit of a probability-of-water-leakage-accidents prediction apparatus according to an embodiment;

Fig. 4    is a block diagram illustrating a functional configuration of a map database unit according to an embodiment;

Fig. 5    is a diagram illustrating an example pipeline map included in buried pipe data according to an embodiment;

Fig. 6    is a diagram illustrating an example data structure of buried pipe attribute data included in buried pipe data according to an embodiment;

Fig. 7    is a diagram illustrating a data structure of a nominal pipe wall thickness database unit;

Fig. 8    is a diagram illustrating an example burial environment classification provisional map;

Fig. 9    is a box plot illustrating a relationship between a burial environment and a corrosion rate;

Fig. 10    is a diagram illustrating a relationship between first geological information and a burial environment;

Fig. 11    is a box plot illustrating a relationship between a specific resistance of groundwater and a degree of corrosion of a buried pipe;

Fig. 12    is a box plot illustrating the relationship between a specific resistance of groundwater in a gravel-based soil and a distance from a coastline;

Fig. 13    is a diagram illustrating types of soils that can be regarded as a gravel-based soil and types of soils that can be regarded as a non-gravel-based soil in a subsurface geological map (small classification);

Fig. 14    is a box plot illustrating a relationship between a specific resistance and an elevation of groundwater in a gravel-based soil at 5 km or less from a coastline;

Fig. 15    is a diagram illustrating an example burial environment classification map;

Fig. 16    is a diagram illustrating a data structure of preprocessed buried pipe data according to an embodiment;

Fig. 17    is a diagram illustrating an example of a probability-of-water-leakage-accidents prediction result according to an embodiment;

Fig. 18    is a diagram illustrating another example of a probability-of-water-leakage-accidents prediction result according to an embodiment;

Fig. 19    is a flowchart illustrating a burial environment classification map creation method according to an embodiment;

Fig. 20    is a flowchart illustrating of steps of creating a burial environment classification map according to an embodiment;

Fig. 21    is a flowchart illustrating of a method of calculating a probability-of-water-leakage-accidents according to an embodiment; and

Fig. 22    is a flowchart illustrating of steps of creating preprocessed buried pipe data according to an embodiment.

Description of embodiments

Hereinafter, an embodiment of the present disclosure will be described. The same components will be denoted by the same reference numerals, and the description thereof will not be repeated.

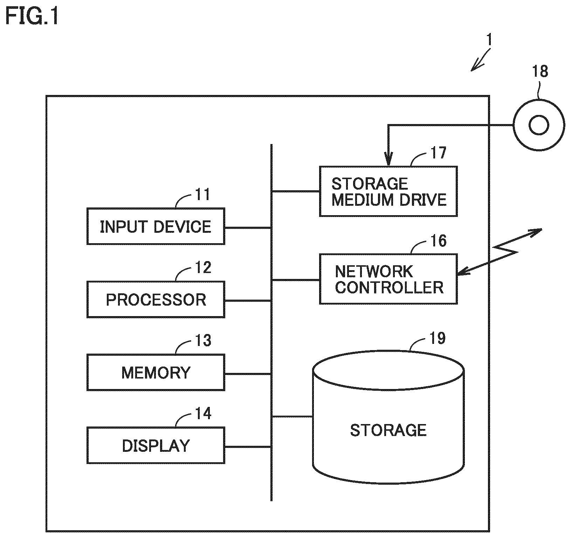

With reference to Figs. 1 to 3, a probability-of-water-leakage-accidents prediction apparatus 1 according to an embodiment will be described as an example corrosion prediction apparatus for a buried pipe. The probability of water leakage accidents of a buried pipe is an example index of corrosion prediction of the buried pipe.

<Hardware Configuration>

With reference to Fig. 1, a hardware configuration of the probability-of-water-leakage-accidents prediction apparatus 1 will be described. The probability-of-water-leakage-accidents prediction apparatus 1 includes an input device 11, a processor 12, a memory 13, a display 14, a network controller 16, a storage medium drive 17, and a storage 19.

The input device 11 accepts various types of input operations. The input device 11 is, for example, a keyboard, a mouse, or a touch panel.

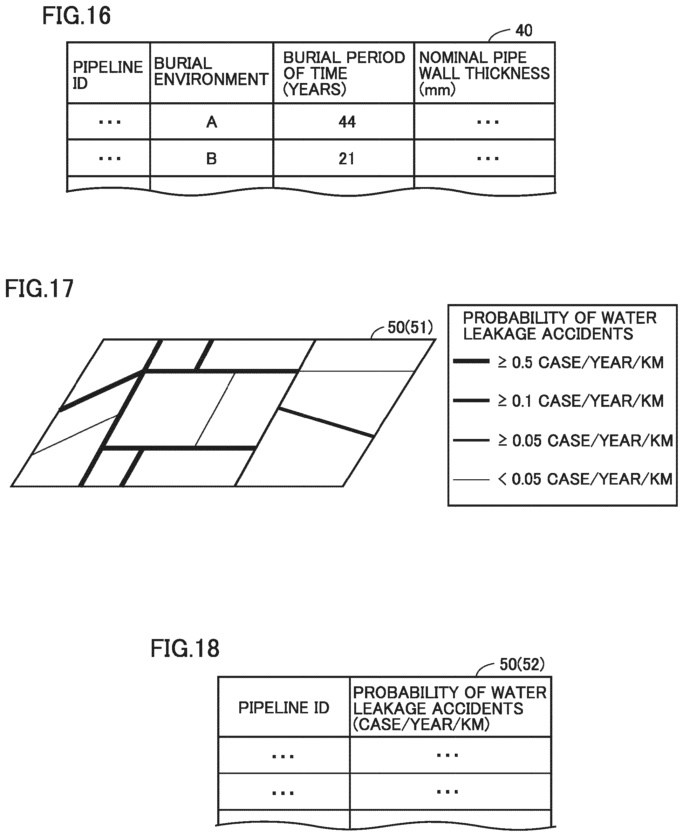

The display 14 displays information or the like required to be processed by the probability-of-water-leakage-accidents prediction apparatus 1. The display 14 displays, for example, a probability-of-water-leakage-accidents prediction result 50 (see Fig. 17 or 18) which will be described later. The display 14 is, for example, an LCD (Liquid Crystal Display) or an organic EL (Electroluminescence) display.

The processor 12 executes a process necessary for implementing the functions of the probability-of-water-leakage-accidents prediction apparatus 1 by executing a program (which will be described later). The processor 12 includes, for example, a CPU, a GPU, or the like.

The memory 13 provides a storage area for temporarily storing program codes or a work memory when the processor 12 executes a program. The memory 13 is, for example, a volatile memory device such as DRAM (Dynamic Random Access Memory) or SRAM (Static Random Access Memory).

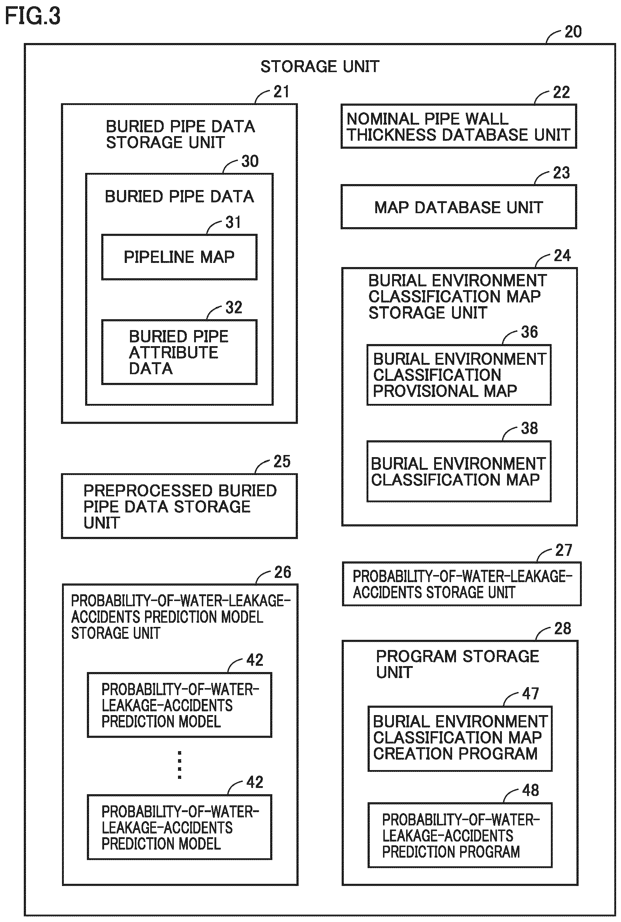

The network controller 16 transmits and receives a program or data to and from an external device (not shown) via a communication network (not shown). For example, the network controller 16 transmits the probability-of-water-leakage-accidents prediction result 50 (see Fig. 17 or 18) to an external device via a communication network. The network controller 16 may receive buried pipe data 30 (see Figs. 3, 5 and 6) from a client via a communication network. The network controller 16 supports any communication system such as Ethernet (registered trademark), wireless LAN, or Bluetooth (registered trademark).

The storage medium drive 17 is a device that reads out a program or data stored in the storage medium 18. The storage medium drive 17 may also be a device that writes a program or data to the storage medium 18.

The storage medium 18 is a non-transitory storage medium, and stores a program or data in a non-volatile manner. The storage medium 18 is, for example, an optical storage medium such as an optical disk (for example, a CD-ROM or a DVD-ROM), a semiconductor storage medium such as a flash memory or a USB memory, a magnetic storage medium such as a hard disk, a floppy disk (FD) or a storage tape, or a magneto-optical storage medium such as a magneto-optical (MO) disk.

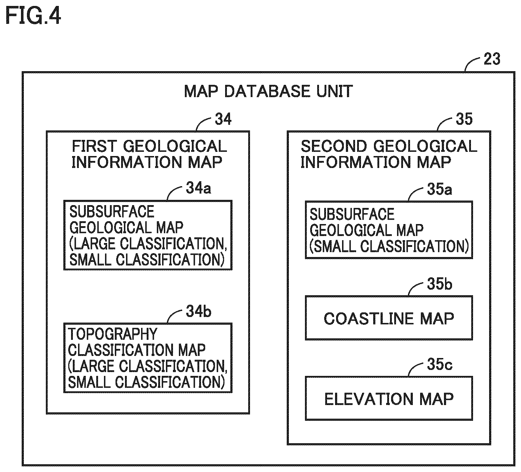

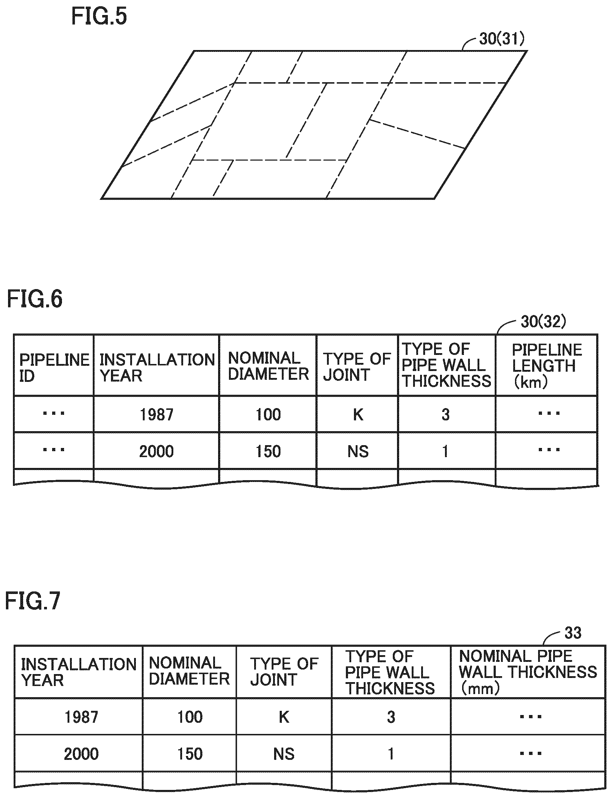

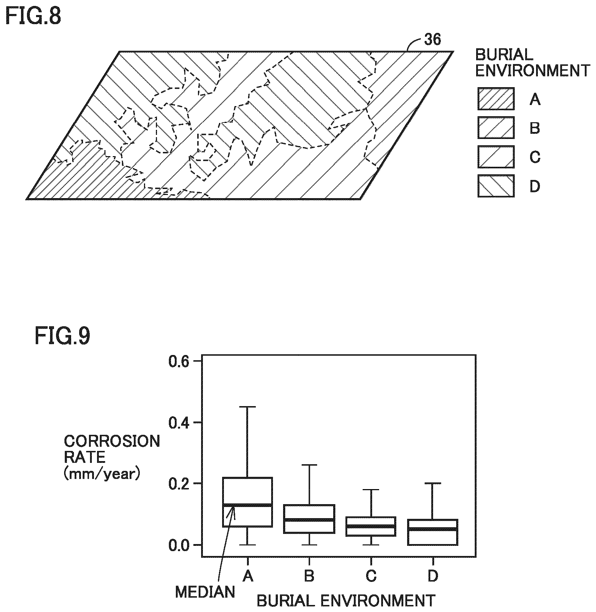

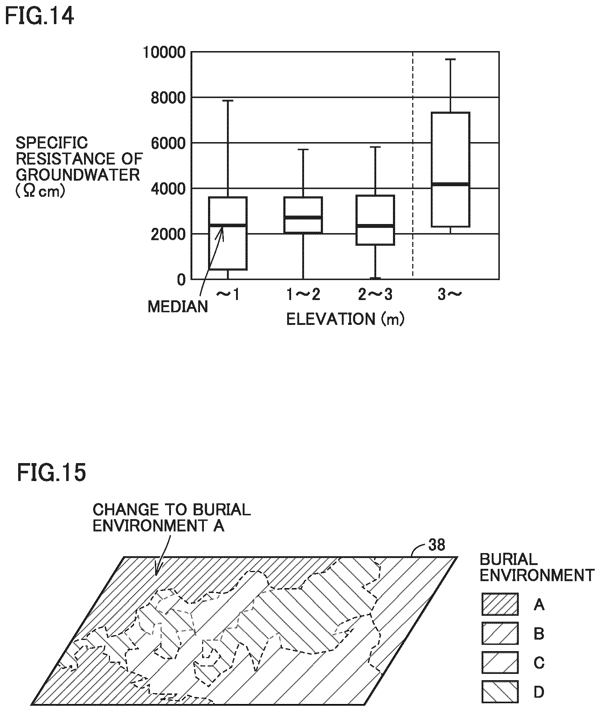

The storage 19 is, for example, a nonvolatile memory device such as a hard disk or a solid state drive (SSD). The storage 19 stores buried pipe data 30 (see Figs. 5 and 6), a geological information map (a first geological information map 34 and a second geological information map 35 illustrated in Fig. 4), a burial environment classification provisional map 36 (see Fig. 8), a burial environment classification map 38 (see Fig. 15), nominal pipe wall thickness data 33 (see Fig. 7), preprocessed buried pipe data 40 (see Fig. 16), a probability-of-water-leakage-accidents prediction model 42 (see Fig. 3), a probability-of-water-leakage-accidents of the buried pipe, programs to be executed in the processor 12, and the like. The programs include a burial environment classification map creation program 47 (see Fig. 3) and a probability-of-water-leakage-accidents prediction program 48 (see Fig. 3).

The burial environment classification map creation program 47 is a program for creating the burial environment classification map 38 (see Fig. 15) from a generally available geological information map (a first geological information map 34 and a second geological information map 35 illustrated in Fig. 4). The probability-of-water-leakage-accidents prediction program 48 is a program for calculating a probability of water leakage accidents of a buried pipe from the buried pipe data 30.

The programs for implementing the functions of the probability-of-water-leakage-accidents prediction apparatus 1, such as the burial environment classification map creation program 47 and the probability-of-water-leakage-accidents prediction program 48, may be stored in the non-transitory storage medium 18 for distribution, and may be installed in the storage 19. The programs for implementing the functions of the probability-of-water-leakage-accidents prediction apparatus 1, such as the burial environment classification map creation program 47 and the probability-of-water-leakage-accidents prediction program 48, may be downloaded to the probability-of-water-leakage-accidents prediction apparatus 1 via the Internet or an intranet.

In the present embodiment, it is described that a general-purpose computer (processor 12) implements the functions of the probability-of-water-leakage-accidents prediction apparatus 1 by executing a program. All or a part of the functions of the probability-of-water-leakage-accidents prediction apparatus 1 may be implemented by using an integrated circuit such as an application specific integrated circuit (ASIC) or a field programmable gate array (FPGA).

<Functional Configuration>

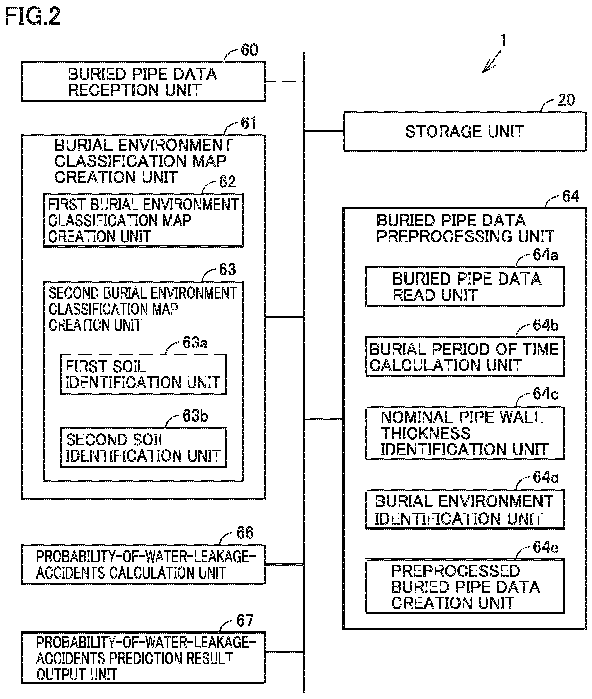

An example functional configuration of the probability-of-water-leakage-accidents prediction apparatus 1 will be described with reference to Figs. 2 to 4. With reference to Fig. 2, the probability-of-water-leakage-accidents prediction apparatus 1 includes a storage unit 20, a buried pipe data reception unit 60, a burial environment classification map creation unit 61, a buried pipe data preprocessing unit 64, a probability-of-water-leakage-accidents calculation unit 66, and a probability-of-water-leakage-accidents prediction result output unit 67.

<Storage Unit 20>

The storage unit 20 is implemented by the storage 19 (see Fig. 1) or the storage medium 18 (see Fig. 1). As illustrated in Fig. 3, the storage unit 20 includes a buried pipe data storage unit 21, a nominal pipe wall thickness database unit 22, a map database unit 23, a burial environment classification map storage unit 24, a preprocessed buried pipe data storage unit 25, a probability-of-water-leakage-accidents prediction model storage unit 26, a probability-of-water-leakage-accidents storage unit 27, and a program storage unit 28.

With reference to Fig. 3, the buried pipe data storage unit 21 stores the buried pipe data 30. The buried pipe data 30 is data about a buried pipe received from a client. The buried pipe is, for example, a water pipe. The buried pipe is buried in the soil. The buried pipe data 30 includes, for example, a pipeline map 31 (see Fig. 5) and buried pipe attribute data 32 (see Fig. 6).

As illustrated in Fig. 5, the pipeline map 31 includes a pipeline ID and a buried location (position) of a buried pipe. In the pipeline map 31, the pipeline ID and the buried location of a buried pipe are associated with each other, and the buried location of a buried pipe is displayed on the map for each pipeline ID of the buried pipe.

With reference to Fig. 6, the buried pipe attribute data 32 includes, for example, a pipeline ID, an installation year, a nominal diameter, a type of joint, a type of pipe wall thickness, and a pipeline length of buried pipes. In the buried pipe attribute data 32, the pipeline ID, the installation year, the nominal diameter, the type of joint, the type of pipe wall thickness, and the pipeline length are associated with each other. The installation year of a buried pipe is the year in which the buried pipe is installed (buried). The type of joint includes type A, type K, type T, and type NS. The type of pipe wall thickness includes type 1, type 2, type 3, or the like. The pipeline length is the length of buried pipes.

With reference to Fig. 3, the nominal pipe wall thickness database unit 22 stores the nominal pipe wall thickness data 33 (see Fig. 7). The nominal pipe wall thickness data 33 includes, for example, an installation year, a nominal diameter, a type of joint, a type of pipe wall thickness, and a nominal pipe wall thickness of a buried pipe. In the nominal pipe wall thickness data 33, the installation year, the nominal diameter, the type of joint, the type of pipe wall thickness, and the nominal pipe wall thickness of a buried pipe are associated with each other. The nominal pipe wall thickness refer to the standard pipe wall thickness.

With reference to Figs. 3 and 4, the map database unit 23 stores the first geological information map 34 and the second geological information map 35. The first geological information map 34 includes, for example, a subsurface geological map (large classification, small classification) 34a and a topography classification map (large classification, small classification) 34b. The subsurface geological map (large classification, small classification) 34a and the topography classification map (large classification, small classification) 34b are provided by a public organization such as the Ministry of Land, Infrastructure, Transport and Tourism of Japan, and are generally available. The second geological information map 35 includes, for example, a subsurface geological map (small classification) 35a, a coastline map 35b, and an elevation map 35c. The subsurface geological map (small classification) 35a, the coastline map 35b, and the elevation map 35c are provided by a public organization such as the Ministry of Land, Infrastructure, Transport and Tourism of Japan, and are generally available. The classification of the subsurface geological map (small classification) 35a corresponds to the small classification of the subsurface geological map (large classification, small classification) 34a.

With reference to Fig. 3, the burial environment classification map storage unit 24 stores the burial environment classification provisional map 36 (see Fig. 8) and the burial environment classification map 38 (see Fig. 15). The burial environment classification map 38 is, for example, a map indicating the location of each of the burial environments A, B, C, and D.

The burial environments A to D each refer to a class of soil in which a buried pipe is buried. In the burial environment classification provisional map 36 and the burial environment classification map 38, the soil in which a buried pipe is buried is classified into four burial environments A to D according to the type of soil, the soil resistivity or the like. The burial environment A represents a soil having a specific resistance of less than 1500 Ω·cm or a soil having a corrosivity to a buried pipe which is equivalent to that of the aforementioned soil. The burial environment B represents a clay soil having a specific resistance of 1500 Ω·cm or more or a soil having a corrosivity to a buried pipe which is equivalent to that of the clay soil. The burial environment C represents a silty soil having a specific resistance of 1500 Ω·cm or more or a soil having a corrosivity to a buried pipe which is equivalent to that of the silty soil. The burial environment D represents a sandy soil having a specific resistance of 1500 Ω·cm or more or a soil having a corrosivity to a buried pipe which is equivalent to that of the sandy soil. Among the burial environments A to D, the burial environment A has the highest corrosivity to the buried pipe.

The soil in which a buried pipe is buried is classified into four burial environments A to D due to the following two reasons. The first reason is that the inventors of the present application have found that there is a statistically significant correlation between the burial environments A to D and the corrosion rate of a buried pipe based on survey data on the relationship between the soil in which a buried pipe is buried and the degree of corrosion of the buried pipe at about 6000 locations throughout Japan (see Fig. 9). The second reason is that the number of survey data concerning the four burial environments A to D accounts for the majority (80% or more) of the total number of the survey data.

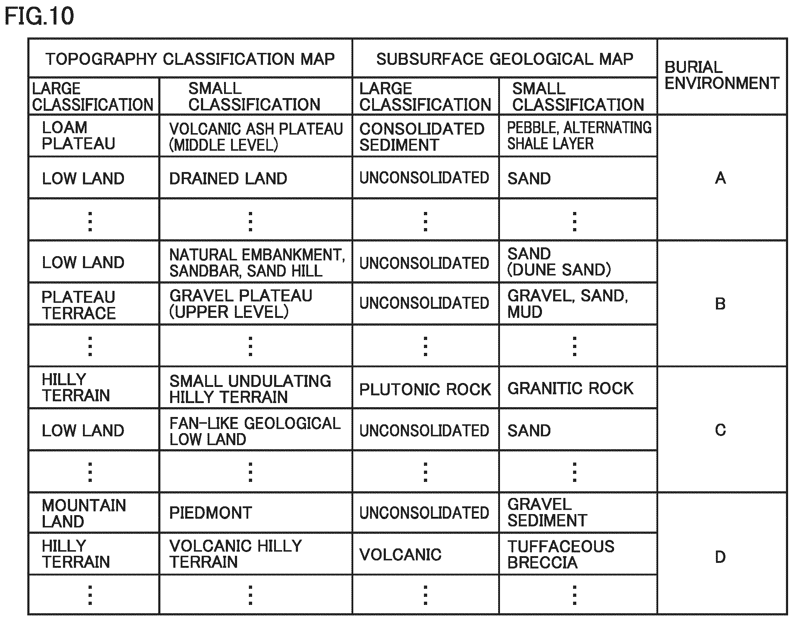

With reference to Figs. 3 and 8, the burial environment classification provisional map 36 is created from, for example, the first geological information map 34. The inventors of the present application have found that there is a statistically significant correlation between the burial environments A to D and a combination (see Fig. 10) of classifications on the subsurface geological map (large classification, small classification) 34a and classifications on the topography classification map (large classification, small classification) 34b with respect to the degree of corrosion of a buried pipe. As illustrated in Fig. 10, the soil in which a buried pipe is buried is classified into four burial environments A to D based on the combination of classifications on the subsurface geological map (large classification, small classification) 34a and classifications on the topography classification map (large classification, small classification) 34b. Thus, the burial environment classification provisional map 36 is created.

With reference to Figs. 3 and 15, the burial environment classification map 38 is created from the burial environment classification provisional map 36 and the second geological information map 35.

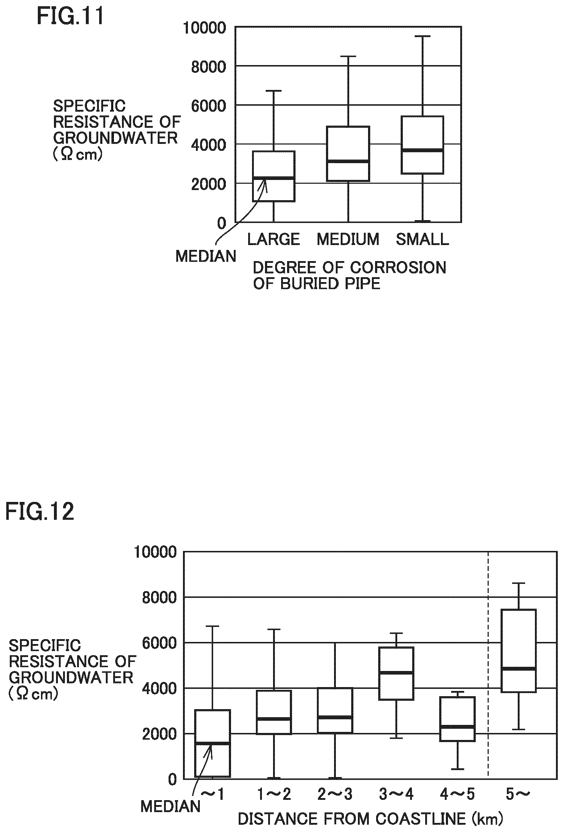

The inventors of the present application have analyzed survey data of certain municipalities with a coastline. The inventors of the present application have found that there is a statistically significant correlation between the specific resistance of groundwater contained in the soil in which a buried pipe is buried and the degree of corrosion of the buried pipe (see Fig. 11). Specifically, the inventors of the present application have found that the lower the specific resistance of groundwater is, the greater the degree of corrosion of the buried pipe will be. In Fig. 11, the degree of corrosion of a buried pipe is large means that the corrosion depth of the buried pipe is 2 mm or more, the degree of corrosion of a buried pipe is medium means that the corrosion depth of the buried pipe is greater than 0 mm and less than 2 mm, and the degree of corrosion of a buried pipe is small means that the corrosion depth of the buried pipe is 0 mm.

With reference to Fig. 12, based on the specific resistance of groundwater contained in a soil that can be regarded as the gravel-based soil in the subsurface geological map (small classification) 35a (see Fig. 4), the location where a buried pipe is buried in the soil, and the coastline map 35b (see Fig. 4) included in the survey data of the municipalities mentioned above, the inventors of the present application have found that in a soil that can be regarded as the gravel-based soil in the subsurface geological map (small classification) 35a, there is a statistically significant correlation (see Fig. 12) between the specific resistance of groundwater and the distance from the coastline to the location where a buried pipe is buried. For example, the inventors of the present application have found that the specific resistance of groundwater contained in the gravel-based soil with a distance of 5 km or less from the coastline is statistically and significantly smaller than the specific resistance of groundwater contained in the gravel-based soil with a distance of more than 5 km from the coastline. A first reason therefor may be that when the soil in which a buried pipe is buried can be regarded as the gravel-based soil, the soil allows groundwater to pass therethrough easily. A second reason therefor may be that the closer the soil in which the buried pipe is buried is to the coastline, the stronger the groundwater contained in the soil is affected by the salt contained in the seawater.

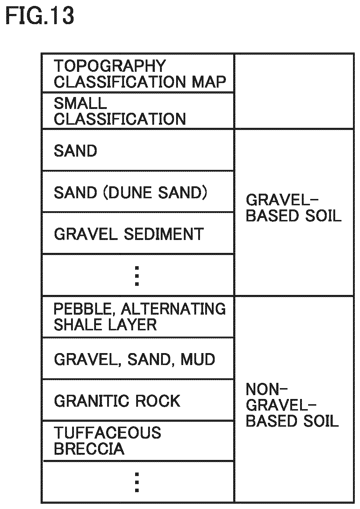

As the sort of soil that can be regarded as the gravel-based soil in the subsurface geological map (small classification) 35a, Fig. 13 illustrates sand, sand (dune sand), gravel sediment or the like as examples in the subsurface geological map (small classification) 35a. On the other hand, as the sort of soil that can be regarded as the non-gravel based soil in the subsurface geological map (small classification) 35a, Fig. 13 illustrates pebble, alternating shale layer, gravel, sand, mud, granitic rock, tuffaceous breccia and the like as examples in the subsurface geological map (small classification) 35a.

Among the survey data of the municipalities mentioned above, the inventors of the present application further analyzed survey data (hereinafter, this data will be referred to as "near-coast survey data") of buried pipes buried in the gravel-based soil with a distance of 5 km or less from the coastline. As illustrated in Fig. 14, based on the specific resistance of groundwater contained in a soil that can be regarded as the gravel-based soil in the subsurface geological map (small classification) 35a (see Fig. 4), the buried location of a buried pipe in the soil, and the elevation map 35c (see Fig. 4), the inventors of the present application have found that the specific resistance of the groundwater at the buried location of the buried pipe is statistically significantly smaller if the soil where the buried pipe is buried can be regarded as the gravel-based soil in the subsurface geological map (small classification) 35a, the buried location of the buried pipe has a distance of 5 km or less from the coastline, and the elevation of the soil where the buried pipe is buried is 3 m or less. The reason therefor may be that the lower the elevation of the soil where the buried pipe is buried, the closer the groundwater to the buried pipe.

Therefore, the burial environment classification provisional map 36 is modified based on the second geological information map 35 to create the burial environment classification map 38. Specifically, a soil permeable to groundwater (groundwater permeable soil) is identified in the burial environment classification provisional map 36 with reference to the burial environment classification provisional map 36 and the subsurface geological map (small classification) 35a (see Fig. 4). The groundwater permeable soil is, for example, a soil that can be regarded as the gravel-based soil in the subsurface geological map (small classification) 35a regardless of the combination of classifications on the subsurface geological map (large classification, small classification) 34a and classifications on the topography classification map (large classification, small classification) 34b (see Fig. 10). A soil which is located within a predetermined distance from the salt-containing water source and has an elevation within a predetermined height is identified from the groundwater permeable soil as a highly corrosive soil with reference to the coastline map 35b and the elevation map 35c. The highly corrosive soil is, for example, a soil in the groundwater permeable soil which has a distance of 5 km or less from the coastline and an elevation of 3 m or less. The highly corrosive soil in the burial environment classification provisional map 36 is identified as the burial environment A having the highest corrosivity to a buried pipe among the burial environments A to D regardless of the burial environment in the burial environment classification provisional map 36. Thus, the burial environment classification map 38 is created.

With reference to Figs. 3 and 16, the preprocessed buried pipe data storage unit 25 stores preprocessed buried pipe data 40. The preprocessed buried pipe data 40 is obtained by processing the buried pipe data 30 (Figs. 3, 5 and 6) with the buried pipe data preprocessing unit 64 (see Fig. 2). The preprocessed buried pipe data 40 includes, for example, a pipeline ID of a buried pipe, a burial environment in the burial environment classification map 38, a burial period of time, and a nominal pipe wall thickness. The burial period of time is a period during which the buried pipe is buried.

With reference to Fig. 3, the probability-of-water-leakage-accidents prediction model storage unit 26 stores a plurality of probability-of-water-leakage-accidents prediction models 42 which are different from each other for a plurality of burial environments. The plurality of probability-of-water-leakage-accidents prediction models 42 include, for example, a probability-of-water-leakage-accidents prediction model for the burial environment A, a probability-of-water-leakage-accidents prediction model for the burial environment B, a probability-of-water-leakage-accidents prediction model for the burial environment C, and a probability-of-water-leakage-accidents prediction model for the burial environment D. The plurality of probability-of-water-leakage-accidents prediction models 42 are not particularly limited, and may be, for example, a probability-of-water-leakage-accidents prediction model disclosed in Japanese Patent Laying-Open No. 2021-56224, or may be a probability-of-water-leakage-accidents estimation formula for a buried pipe provided by the Water Research Center of Japan.

The probability-of-water-leakage-accidents estimation formula for a buried pipe provided by the Water Research Center of Japan is given by the following equation (1). Where y represents a probability of water leakage accidents (case/km/year) of a buried pipe, $C_1$ represents a correction coefficient for the pipe specification, $C_2$ represents a correction coefficient for the pipe diameter, $C_3$ represents a correction coefficient for the conditions of ground where the buried pipe is buried, and f(T) represents the standard accident rate curve for each pipe type. Here, f(T) is given by the following equation (2). Where T represents a buried period of a buried pipe, and coefficients a and b represent the degree of increase in the probability of water leakage accidents for each pipe type over time.

$$Y = C_1 \cdot C_2 \cdot C_3 \cdot f(T) \qquad (1)$$

$$f(T) = a \cdot T^b \qquad (2)$$

With reference to Fig. 3, the probability-of-water-leakage-accidents storage unit 27 stores probability of water leakage accidents of a buried pipe calculated by the probability-of-water-leakage-accidents calculation unit 66 (see Fig. 2). As illustrated in Fig. 18, the probability of water leakage accidents of a buried pipe is stored in the probability-of-water-leakage-accidents storage unit 27 in association with the pipeline ID of the buried pipe.

With reference to Fig. 3, the program storage unit 28 stores programs (for example, a burial environment classification map creation program 47 and a probability-of-water-leakage-accidents prediction program 48) for implementing the functions of the probability-of-water-leakage-accidents prediction apparatus 1.

<Buried pipe Data Reception Unit 60>

With reference to Fig. 2, the buried pipe data reception unit 60 receives the buried pipe data 30 (see Figs. 3, 5, and 6) from a client. The buried pipe data 30 is stored in the buried pipe data storage unit 21 (see Fig. 3). The buried pipe data 30 may be stored in the storage medium 18 (see Fig. 1) provided by the client. The buried pipe data 30 may be received from the client via a communication network. The buried pipe data 30 may be stored in the storage 19 (see Fig. 1) in advance.

<Burial Environment Classification Map Creation Unit 61>

The burial environment classification map creation unit 61 includes a first burial environment classification map creation unit 62 and a second burial environment classification map creation unit 63.

The first burial environment classification map creation unit 62 creates a burial environment classification provisional map 36 (see Figs. 3 and 8) from the first geological information map 34 (see Fig. 4). The first geological information map 34 includes, for example, a subsurface geological map (large classification, small classification) 34a and a topography classification map (large classification, small classification) 34b.

Specifically, the first burial environment classification map creation unit 62 reads the first geological information map 34 from the map database unit 23. The first burial environment classification map creation unit 62 classifies the soil in which a buried pipe is buried into four burial environments A to D based on a combination of classifications on the subsurface geological map (large classification, small classification) 34a and classifications on the topography classification map (large classification, small classification) 34b. Thus, the first burial environment classification map creation unit 62 creates the burial environment classification provisional map 36. The first burial environment classification map creation unit 62 outputs the burial environment classification provisional map 36 to the burial environment classification map storage unit 24 (see Fig. 3).

The second burial environment classification map creation unit 63 creates a burial environment classification map 38 from the burial environment classification provisional map 36 and the second geological information map 35 (see Fig. 4). The second burial environment classification map creation unit 63 includes a first soil identification unit 63a and a second soil identification unit 63b. The second geological information map 35 includes, for example, a subsurface geological map (small classification) 35a, a coastline map 35b, and an elevation map 35c.

Specifically, the first soil identification unit 63a reads the burial environment classification provisional map 36 from the burial environment classification map storage unit 24, and reads the subsurface geological map (small classification) 35a (see Fig. 4) from the map database unit 23. The first soil identification unit 63a identifies a soil permeable to groundwater (groundwater permeable soil) from the burial environment classification provisional map 36. The groundwater permeable soil is, for example, a soil that can be regarded as the gravel-based soil in the subsurface geological map (small classification) 35a in the burial environment classification provisional map 36.

The second soil identification unit 63b reads the coastline map 35b and the elevation map 35c from the map database unit 23. The second soil identification unit 63b identifies, from the groundwater permeable soil identified by the first soil identification unit 63a, a soil which is located within a predetermined distance from the salt-containing water source and has an elevation within a predetermined height as the highly corrosive soil. For example, the second soil identification unit 63b identifies, from the groundwater permeable soil, a soil which has a distance of 5 km or less from the coastline and an elevation of 3 m or less as the highly corrosive soil. The second soil identification unit 63b identifies the highly corrosive soil from the burial environment classification provisional map 36 as the burial environment A having the highest corrosivity to a buried pipe among the burial environments A to D regardless of the burial environment in the burial environment classification provisional map 36. Thus, the second burial environment classification map creation unit 63 creates the burial environment classification map 38. The second burial environment classification map creation unit 63 outputs the burial environment classification map 38 to the burial environment classification map storage unit 24 (see Fig. 3).

<Buried pipe Data Preprocessing Unit 64>

With reference to Fig. 2, the buried pipe data preprocessing unit 64 creates the preprocessed buried pipe data 40 (see Fig. 16) from the buried pipe data 30 (see Figs. 3, 5 and 6). The buried pipe data preprocessing unit 64 includes a buried pipe data read unit 64a, a burial period of time calculation unit 64b, a nominal pipe wall thickness identification unit 64c, a burial environment identification unit 64d, and a preprocessed buried pipe data creation unit 64e.

Specifically, the buried pipe data read unit 64a reads the buried pipe data 30 from the buried pipe data storage unit 21 (see Fig. 3). The buried pipe data 30 includes, for example, a pipeline map 31 (see Fig. 5) and buried pipe attribute data 32 (see Figs. 3 and 6).

The burial period of time calculation unit 64b calculates the burial period of time of a buried pipe for each pipeline ID. For example, in order to obtain a prediction result of the probability of water leakage accidents of a buried pipe in the current year (the year in which the probability of water leakage accidents of the buried pipe is predicted), the burial period of time calculation unit 64b calculates a difference between the current year stored in the storage unit 20 and the installation year (see Fig. 6) in the buried pipe attribute data 32 as the burial period of time of the buried pipe (see Fig. 16). In order to obtain a prediction result of the probability of water leakage accidents of a buried pipe in a future year, the burial period of time calculation unit 64b calculates a difference between the future year received by the input device 11 (see Fig. 1) and stored in the storage unit 20 and the installation year (see Fig. 6) in the buried pipe attribute data 32 as the burial period of time (see Fig. 16) of the buried pipe.

The nominal pipe wall thickness identification unit 64c reads the nominal pipe wall thickness data 33 (see Fig. 7) from the nominal pipe wall thickness database unit 22 (see Fig. 3). The nominal pipe wall thickness identification unit 64c identifies the nominal pipe wall thickness of a buried pipe for each pipeline ID with reference to the installation year, the nominal diameter, the type of joint, and the type of pipe wall thickness (see Fig. 6) in the buried pipe attribute data 32 and the nominal pipe wall thickness data 33.

The burial environment identification unit 64d reads the burial environment classification map 38 (see Fig. 15) from the burial environment classification map storage unit 24 (see Fig. 3). The burial environment identification unit 64d identifies a burial environment of a buried pipe for each pipeline ID with reference to the pipeline map 31 and the burial environment classification map 38. The preprocessed buried pipe data creation unit 64e creates the preprocessed the buried pipe data 40 (see Fig. 16) by combining the burial period of time, the nominal pipe wall thickness, and the burial environment of a buried pipe for each pipeline ID. The buried pipe data preprocessing unit 64 outputs the preprocessed buried pipe data 40 to the preprocessed buried pipe data storage unit 25 (see Fig. 3).

<Probability-of-water-leakage-accidents Calculation Unit 66>

With reference to Figs. 2 and 3, the probability-of-water-leakage-accidents calculation unit 66 calculates the probability of water leakage accidents of a buried pipe for each pipeline ID by applying the preprocessed buried pipe data 40 to any of the plurality of probability-of-water-leakage-accidents prediction models 42 stored in the probability-of-water-leakage-accidents prediction model storage unit 26.

Specifically, the probability-of-water-leakage-accidents calculation unit 66 reads the preprocessed buried pipe data 40 from the preprocessed buried pipe data storage unit 25. The probability-of-water-leakage-accidents calculation unit 66 reads a burial environment for each pipeline ID from the preprocessed buried pipe data 40. The probability-of-water-leakage-accidents calculation unit 66 reads a read probability-of-water-leakage-accidents prediction model 42 for the burial environment from the plurality of probability-of-water-leakage-accidents prediction models 42 stored in the probability-of-water-leakage-accidents prediction model storage unit 26 (see Fig. 3). The probability-of-water-leakage-accidents calculation unit 66 inputs the burial period of time and the nominal pipe wall thickness for each pipeline ID to the probability-of-water-leakage-accidents prediction model 42, and calculates the probability of water leakage accidents of a buried pipe for each pipeline ID. The probability of water leakage accidents of a buried pipe is the number of water leakage accidents of the buried pipe per unit time (for example, 1 year) and per unit distance (for example, 1 km). The probability-of-water-leakage-accidents calculation unit 66 outputs the probability of water leakage accidents of the buried pipe for each pipeline ID to the probability-of-water-leakage-accidents storage unit 27 (see Fig. 3).

<Probability-of-water-leakage-accidents Prediction Result Output Unit 67>

With reference to Fig. 2, the probability-of-water-leakage-accidents prediction result output unit 67 outputs a probability-of-water-leakage-accidents prediction result 50 of the buried pipe to at least one of the display 14, the storage medium 18 or the storage 19 illustrated in Fig. 1. The probability-of-water-leakage-accidents prediction result 50 may be, for example, a probability-of-water-leakage-accidents prediction map 51 (see Fig. 17) or a probability-of-water-leakage-accidents prediction table 52 (see Fig. 18).

The probability-of-water-leakage-accidents prediction result output unit 67 reads the pipeline map 31 (see Fig. 5) from the buried pipe data storage unit 21 (see Fig. 3). The probability-of-water-leakage-accidents prediction result output unit 67 reads the pipeline ID and the probability of water leakage accidents of a buried pipe from the probability-of-water-leakage-accidents storage unit 27. The probability-of-water-leakage-accidents prediction result output unit 67 creates the probability-of-water-leakage-accidents prediction map 51 by reflecting the probability of water leakage accidents of a buried pipe for each pipeline ID in the pipeline map 31. In the probability-of-water-leakage-accidents prediction map 51, the position and the probability of water leakage accidents of a buried pipe are displayed on the map for each pipeline ID.

The probability-of-water-leakage-accidents prediction result output unit 67 creates the probability-of-water-leakage-accidents prediction table 52 by associating the pipeline ID and the probability of water leakage accidents with each other. In the probability-of-water-leakage-accidents prediction table 52, the pipeline ID and the probability of water leakage accidents of the buried pipe are associated with each other.

<Burial Environment Classification Map Creation Method>

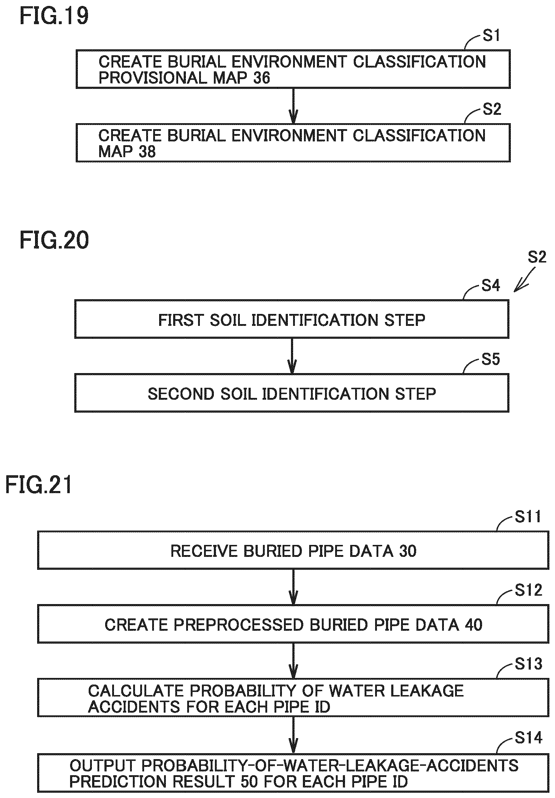

With reference to Figs. 19 and 20, a burial environment classification map creation method according to the present embodiment will be described. The burial environment classification map creation method according to the present embodiment is executed by the burial environment classification map creation unit 61 (see Fig. 2). The burial environment classification map creation method according to the present embodiment includes a step of creating a burial environment classification provisional map 36 (step S1) and a step of creating a burial environment classification map (step S2).

The step of creating a burial environment classification provisional map 36 (step S1) is executed by the first burial environment classification map creation unit 62 (see Fig. 2). In step S1, the first burial environment classification map creation unit 62 creates a burial environment classification provisional map 36 (see Figs. 3 and 8) from the first geological information map 34 (see Fig. 4). The first geological information map 34 includes, for example, a subsurface geological map (large classification, small classification) 34a and a topography classification map (large classification, small classification) 34b.

Specifically, the first burial environment classification map creation unit 62 reads the first geological information map 34 from the map database unit 23. The first burial environment classification map creation unit 62 classifies the soil in which the buried pipe is buried into four burial environments A to D based on a combination of classifications on the subsurface geological map (large classification, small classification) 34a and classifications on the topography classification map (large classification, small classification) 34b. Thus, the first burial environment classification map creation unit 62 creates the burial environment classification provisional map 36. The first burial environment classification map creation unit 62 outputs the burial environment classification provisional map 36 to the burial environment classification map storage unit 24 (see Fig. 3). The burial environment classification provisional map 36 is stored in the burial environment classification map storage unit 24.

The step of creating a burial environment classification map 38 (step S2) is executed by the second burial environment classification map creation unit 63 (see Fig. 2). In step S2, the second burial environment classification map creation unit 63 creates a burial environment classification map 38 (see Figs. 3 and 15) from the burial environment classification provisional map 36 and the second geological information map 35 (see Fig. 4). The second geological information map 35 includes, for example, a subsurface geological map (small classification) 35a, a coastline map 35b, and an elevation map 35c.

As illustrated in Fig. 20, the step of creating a burial environment classification map (step S2) includes a first soil identification step (step S4). Specifically, the first soil identification unit 63a reads the burial environment classification provisional map 36 from the burial environment classification map storage unit 24, and reads the subsurface geological map (small classification) 35a (see Fig. 4) from the map database unit 23. The first soil identification unit 63a identifies, from the burial environment classification provisional map 36, a soil permeable to groundwater (groundwater permeable soil). The groundwater permeable soil is, for example, a soil that can be regarded as the gravel-based soil in the subsurface geological map (small classification) 35a in the burial environment classification provisional map 36.

As illustrated in Fig. 20, the step of creating a burial environment classification map (step S2) further includes a second soil identification step (step S5). Specifically, the second soil identification unit 63b reads the coastline map 35b and the elevation map 35c from the map database unit 23. The second soil identification unit 63b identifies, from the groundwater permeable soil identified by the first soil identification unit 63a, a soil which is located within a predetermined distance from the salt-containing water source and has an elevation within a predetermined height as a highly corrosive soil. For example, the second soil identification unit 63b identifies, from the groundwater permeable soil, a soil which has a distance of 5 km or less from the coastline and an elevation of 3 m or less as the highly corrosive soil. The second soil identification unit 63b identifies the highly corrosive soil from the burial environment classification provisional map 36 as the burial environment A having the highest corrosivity to a buried pipe among the burial environments A to D regardless of the burial environment in the burial environment classification provisional map 36. Thus, the second burial environment classification map creation unit 63 creates the burial environment classification map 38.

The second burial environment classification map creation unit 63 outputs the burial environment classification map 38 to the burial environment classification map storage unit 24 (see Fig. 3). The burial environment classification map 38 is stored in the burial environment classification map storage unit 24.

<Probability-of-water-leakage-accidents Prediction Method>

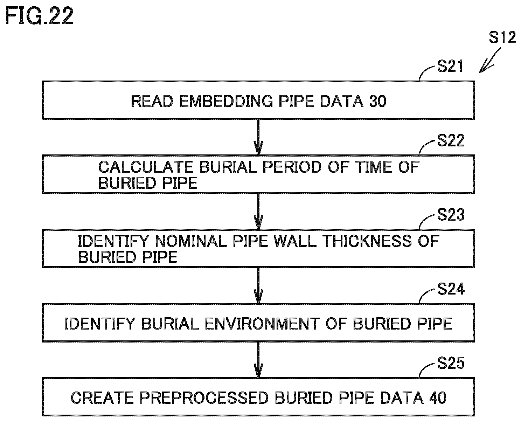

With reference to Figs. 21 and 22, a probability-of-water-leakage-accidents prediction method according to the present embodiment will be described as an example method of predicting a degree of corrosion of a buried pipe.

With reference to Fig. 21, the buried pipe data reception unit 60 (see Fig. 2) receives the buried pipe data 30 (see Figs. 3, 5, and 6) from a client (step S11). The buried pipe data reception unit 60 outputs the buried pipe data 30 to the buried pipe data storage unit 21 (see Fig. 3). The buried pipe data 30 includes, for example, a pipeline map 31 (see Fig. 5) and buried pipe attribute data 32 (see Fig. 6). The buried pipe data 30 is stored in the buried pipe data storage unit 21 (see Fig. 3). The buried pipe data 30 may be stored in the storage medium 18 (see Fig. 1) provided by the client. The buried pipe data 30 may be received from the client via a communication network. The buried pipe data 30 may be stored in the storage 19 (see Fig. 1) in advance.

With reference to Figs. 21 and 22, the buried pipe data preprocessing unit 64 (see Fig. 2) creates the preprocessed buried pipe data 40 (see Fig. 16) from the buried pipe data 30 (see Figs. 3, 5 and 6) stored in the buried pipe data storage unit 21 (see Fig. 3) (step S12).

Specifically, with reference to Fig. 22, the buried pipe data read unit 64a (see Fig. 2) reads the buried pipe data 30 from the buried pipe data storage unit 21 (see Fig. 3) (step S21). The buried pipe data 30 includes, for example, a pipeline map 31 (see Fig. 5) and buried pipe attribute data 32 (see Figs. 3 and 6).

With reference to Fig. 22, the burial period of time calculation unit 64b (see Fig. 2) calculates the burial period of time of a buried pipe for each pipeline ID (step S22). For example, in order to obtain a prediction result of the probability of water leakage accidents of a buried pipe in the current year (the year in which the probability of water leakage accidents of the buried pipe is predicted), the burial period of time calculation unit 64b calculates a difference between the current year stored in the storage unit 20 and the installation year (see Fig. 6) in the buried pipe attribute data 32 as the burial period of time of the buried pipe (see Fig. 16). In order to obtain a prediction result of the probability of water leakage accidents of a buried pipe in a future year, the burial period of time calculation unit 64b calculates a difference between the future year received by the input device 11 (see Fig. 1) and stored in the storage unit 20 and the installation year (see Fig. 6) in the buried pipe attribute data 32 as the burial period of time (see Fig. 16) of the buried pipe.

With reference to Fig. 22, the nominal pipe wall thickness identification unit 64c (see Fig. 2) identifies the nominal pipe wall thickness of a buried pipe for each pipeline ID (step S23). Specifically, the nominal pipe wall thickness identification unit 64c reads the nominal pipe wall thickness data 33 (see Fig. 7) from the nominal pipe wall thickness database unit 22 (see Fig. 3). The nominal pipe wall thickness identification unit 64c identifies the nominal pipe wall thickness of a buried pipe for each pipeline ID with reference to the installation year, the nominal diameter, the type of joint, and the type of pipe wall thickness (see Fig. 6) in the buried pipe attribute data 32 and the nominal pipe wall thickness data 33.

With reference to Fig. 22, the burial environment identification unit 64d (see Fig. 2) identifies the burial environment of a buried pipe for each pipeline ID (step S24). Specifically, the burial environment identification unit 64d reads the burial environment classification map 38 (see Fig. 15) from the burial environment classification map storage unit 24 (see Fig. 3). The burial environment identification unit 64d identifies the burial environment of a buried pipe for each pipeline ID with reference to the pipeline map 31 and the burial environment classification map 38.

With reference to Fig. 22, the preprocessed buried pipe data creation unit 64e (see Fig. 2) creates the preprocessed buried pipe data 40 (see Fig. 16) by combining the burial period of time, the nominal pipe wall thickness, and the burial environment of a buried pipe obtained for each pipeline ID (step S25). The buried pipe data preprocessing unit 64 outputs the preprocessed buried pipe data 40 to the preprocessed buried pipe data storage unit 25 (see Fig. 3). The preprocessed buried pipe data 40 is stored in the preprocessed buried pipe data storage unit 25.

With reference to Fig. 21, the probability-of-water-leakage-accidents calculation unit 66 (see Fig. 2) calculates the probability of water leakage accidents of a buried pipe for each pipeline ID (step S13). Specifically, the probability-of-water-leakage-accidents calculation unit 66 reads the preprocessed buried pipe data 40 from the preprocessed buried pipe data storage unit 25 (see Fig. 3). The probability-of-water-leakage-accidents calculation unit 66 reads the burial environment for each pipeline ID from the preprocessed buried pipe data 40. The probability-of-water-leakage-accidents calculation unit 66 reads a read probability-of-water-leakage-accidents prediction model 42 for the burial environment from the plurality of probability-of-water-leakage-accidents prediction models 42 stored in the probability-of-water-leakage-accidents prediction model storage unit 26 (see Fig. 3).

The probability-of-water-leakage-accidents calculation unit 66 inputs the burial period of time and the nominal pipe wall thickness of the buried pipe for each pipeline ID to the probability-of-water-leakage-accidents prediction model 42, and calculates the probability of water leakage accidents of the buried pipe for each pipeline ID. The probability-of-water-leakage-accidents calculation unit 66 outputs the probability of water leakage accidents of the buried pipe for each pipeline ID to the probability-of-water-leakage-accidents storage unit 27 (see Fig. 3). As illustrated in Fig. 18, the probability of water leakage accidents of the buried pipe is stored in the probability-of-water-leakage-accidents storage unit 27 in association with the pipeline ID.

With reference to Fig. 21, the probability-of-water-leakage-accidents prediction result output unit 67 (see Fig. 2) outputs a probability-of-water-leakage-accidents prediction result 50 to at least one of the display 14, the storage medium 18, or the storage 19 illustrated in Fig. 1 (step S14). The probability-of-water-leakage-accidents prediction result 50 may be, for example, a probability-of-water-leakage-accidents prediction map 51 (see Fig. 17) or a probability-of-water-leakage-accidents prediction table 52 (see Fig. 18).

The probability-of-water-leakage-accidents prediction result output unit 67 reads the pipeline map 31 (see Fig. 5) from the buried pipe data storage unit 21 (see Fig. 3). The probability-of-water-leakage-accidents prediction result output unit 67 reads the pipeline ID and the probability of water leakage accidents of the buried pipe from the probability-of-water-leakage-accidents storage unit 27. The probability-of-water-leakage-accidents prediction result output unit 67 creates the probability-of-water-leakage-accidents prediction map 51 by reflecting the probability of water leakage accidents of the buried pipe for each pipeline ID in the pipeline map 31. The probability-of-water-leakage-accidents prediction result output unit 67 creates the probability-of-water-leakage-accidents prediction table 52 by associating the pipeline ID and the probability of water leakage accidents with each other.

The burial environment classification map creation program 47 (see Fig. 3) causes the processor 12 (see Fig. 1) to execute the burial environment classification map creation method of the present embodiment. The probability-of-water-leakage-accidents prediction program 48 (see Fig. 3) causes the processor 12 (see Fig. 1) to execute the probability-of-water-leakage-accidents prediction method of the present embodiment. The programs such as the burial environment classification map creation program 47 and the probability-of-water-leakage-accidents prediction program 48 may be recorded in a computer-readable recording medium (a non-transitory computer-readable recording medium, for example, the storage medium 18) of the present embodiment.

In the present embodiment, the groundwater permeable soil is not limited to a soil that can be regarded as the gravel-based soil in the subsurface geological map (small classification) 35a (see Fig. 4). In the present embodiment, sea is assumed as the salt-containing water source, but the salt-containing water source may be, for example, a salt lake. The distance of the second region from the coastline is not limited to 5 km or less. The elevation of the second region is not limited to 3 m or less.

The effects of the burial environment classification map creation apparatus (the second burial environment classification map creation unit 63), the burial environment identification apparatus (the buried pipe data preprocessing unit 64), the burial environment classification map creation method, the burial environment identification method, and the program according to the present embodiment will be described.

The burial environment classification map creation apparatus (the second burial environment classification map creation unit 63) according to the present embodiment includes a first soil identification unit 63a that identifies a groundwater permeable soil which is a soil permeable to groundwater, and a second soil identification unit 63b that identifies, from the groundwater permeable soil, a soil which is located within a predetermined distance from a salt-containing water source and has an elevation within a predetermined height as a highly corrosive soil which is highly corrosive to a pipe.

Thus, the highly corrosive soil can be identified more accurately. According to the burial environment classification map creation apparatus of the present embodiment, it is possible to predict a degree of corrosion of a buried pipe more accurately.

The burial environment identification apparatus (the buried pipe data preprocessing unit 64) of the present embodiment includes a buried pipe data read unit 64a that reads buried pipe data 30 including a position of a buried pipe, and a burial environment identification unit 64d that identifies a burial environment of the buried pipe by applying the buried pipe data 30 to a burial environment classification map 38 created by the burial environment classification map creation apparatus (the second burial environment classification map creation unit 63) of the present embodiment.

Therefore, the burial environment of the buried pipe can be identified more accurately. According to the burial environment identification apparatus of the present embodiment, it is possible to predict a degree of corrosion of a buried pipe more accurately.

The burial environment classification map creation method according to the present embodiment includes a step of identifying a groundwater permeable soil which is a soil permeable to groundwater (step S4), and a step of identifying, from the groundwater permeable soil, a soil which is located within a predetermined distance from a salt-containing water source and has an elevation within a predetermined height as a highly corrosive soil which is highly corrosive to a pipe (step S5).

Thus, the highly corrosive soils can be identified more accurately. According to the burial environment classification map creation method of the present embodiment, it is possible to more accurately predict a degree of corrosion of a buried pipe.

The burial environment identification method of the present embodiment includes a step of reading buried pipe data 30 including a position of a buried pipe (step S21), and a step of identifying a burial environment of the buried pipe by applying the buried pipe data 30 to the burial environment classification map 38 created by the burial environment classification map creation method of the present embodiment (step S24).

Therefore, the burial environment of the buried pipe can be identified more accurately. According to the burial environment identification method of the present embodiment, it is possible to more accurately predict a degree of corrosion of a buried pipe.

The program of the present embodiment causes the processor to execute each step of the burial environment classification map creation method of the present embodiment.

Thus, highly corrosive soils can be identified more accurately. According to the program of the present embodiment, it is possible to more accurately predict a degree of corrosion of a buried pipe.

The program of the present embodiment causes a processor to execute each step of the burial environment identification method of the present embodiment.

Thus, the burial environment of the buried pipe can be identified more accurately. According to the program of the present embodiment, it is possible to more accurately predict a degree of corrosion of a buried pipe.

It should be understood that the embodiments disclosed herein are illustrative and non-restrictive in all respects. The scope of the present invention is defined by the terms of the claims rather than the description of the embodiments above, and is intended to include any modifications within the scope and meaning equivalent to the terms of the claims.

Reference sign list:

1: probability-of-water-leakage-accidents prediction apparatus; 11: input device; 12: processor; 13: memory; 14: display; 16: network controller; 17: storage medium drive; 18: storage medium; 19: storage; 20: storage unit; 21: buried pipe data storage unit; 22: nominal pipe wall thickness database unit; 23: map database unit; 24: burial environment classification map storage unit; 25: preprocessed buried pipe data storage unit; 26: probability-of-water-leakage-accidents prediction model storage unit; 27: probability-of-water-leakage-accidents storage unit; 28: program storage unit;

30: buried pipe data; 31: pipeline map; 32: buried pipe attribute data; 33: nominal pipe wall thickness data; 34: first geological information map; 34a: subsurface geological map (large classification, small classification); 34b: topography classification map (large classification, small classification); 35: second geological information map; 35a: subsurface geological map (small classification); 35b: coastline map; 35c: elevation map; 36: burial environment classification provisional map; 38: burial environment classification map;

40: preprocessed buried pipe data; 42: probability-of-water-leakage-accidents prediction model; 47: burial environment classification map creation program; 48: probability-of-water-leakage-accidents prediction program;

50: probability-of-water-leakage-accidents prediction result; 51: probability-of-water-leakage-accidents prediction map; 52: probability-of-water-leakage-accidents prediction table;

60: buried pipe data reception unit; 61: burial environment classification map creation unit; 62: first burial environment classification map creation unit; 63: second burial environment classification map creation unit; 63a: first soil identification unit; 63b: second soil identification unit; 64: buried pipe data preprocessing unit; 64a: buried pipe data read unit; 64b: burial period of time calculation unit; 64c: nominal pipe wall thickness identification unit; 64d: burial environment identification unit; 64e: preprocessed buried pipe data creation unit; 66: probability-of-water-leakage-accidents calculation unit; 67: probability-of-water-leakage-accidents prediction result output unit.

**Claims**

1. A burial environment classification map creation apparatus comprising:

    - a first soil identification unit that identifies a groundwater permeable soil which is a soil permeable to groundwater; and
- a second soil identification unit that identifies, from the groundwater permeable soil, a soil which is located within a predetermined distance from a salt-containing water source and has an elevation within a predetermined height as a highly corrosive soil which is highly corrosive to a pipe.

2. A burial environment identification apparatus comprising:

- a buried pipe data read unit that reads buried pipe data including a position of a buried pipe; and
- a burial environment identification unit that identifies a burial environment of the buried pipe by applying the buried pipe data to a burial environment classification map created by the burial environment classification map creation apparatus according to claim 1.

3. A burial environment classification map creation method comprising:

- a step of identifying a groundwater permeable soil which is a soil permeable to groundwater; and
- a step of identifying, from the groundwater permeable soil, a soil which is located within a predetermined distance from a salt-containing water source and has an elevation within a predetermined height as a highly corrosive soil which is highly corrosive to a pipe.

4. A burial environment identification method comprising:

- a step of reading buried pipe data including a position of a buried pipe; and
- a step of identifying a burial environment of the buried pipe by applying the buried pipe data to a burial environment classification map created by the burial environment classification map creation method according to claim 3.

5. A program that causes a processor to execute each step of the burial environment classification map creation method according to claim 3.

6. A program that causes a processor to execute each step of the burial environment identification method according to claim 4.

FIG.1

FIG.2

```
┌─────────────────────────────┐ ⌐60
│   BURIED PIPE DATA          │
│   RECEPTION UNIT            │
└─────────────────────────────┘

┌─────────────────────────────┐ ⌐61
│ BURIAL ENVIRONMENT          │
│ CLASSIFICATION MAP          │
│ CREATION UNIT       ⌐62     │
│ ┌─────────────────────────┐ │
│ │FIRST BURIAL ENVIRONMENT │ │
│ │CLASSIFICATION MAP       │ │
│ │CREATION UNIT            │ │
│ └─────────────────────────┘ │
│                     ⌐63     │
│ ┌─────────────────────────┐ │
│ │SECOND BURIAL ENVIRONMENT│ │
│ │CLASSIFICATION MAP       │ │
│ │CREATION UNIT            │ │
│ │            ⌐63a         │ │
│ │ ┌─────────────────────┐ │ │
│ │ │FIRST SOIL           │ │ │
│ │ │IDENTIFICATION UNIT  │ │ │
│ │ └─────────────────────┘ │ │
│ │            ⌐63b         │ │
│ │ ┌─────────────────────┐ │ │
│ │ │SECOND SOIL          │ │ │
│ │ │IDENTIFICATION UNIT  │ │ │
│ │ └─────────────────────┘ │ │
│ └─────────────────────────┘ │
└─────────────────────────────┘
```

1

```
┌─────────────────────────────┐ ⌐20
│   STORAGE UNIT              │
└─────────────────────────────┘
```

```
┌─────────────────────────────┐ ⌐64
│ BURIED PIPE DATA            │
│ PREPROCESSING UNIT          │
│                     ⌐64a    │
│ ┌─────────────────────────┐ │
│ │BURIED PIPE DATA         │ │
│ │READ UNIT                │ │
│ └─────────────────────────┘ │
│                     ⌐64b    │
│ ┌─────────────────────────┐ │
│ │BURIAL PERIOD OF TIME    │ │
│ │CALCULATION UNIT         │ │
│ └─────────────────────────┘ │
│                     ⌐64c    │
│ ┌─────────────────────────┐ │
│ │NOMINAL PIPE WALL        │ │
│ │THICKNESS                │ │
│ │IDENTIFICATION UNIT      │ │
│ └─────────────────────────┘ │
│                     ⌐64d    │
│ ┌─────────────────────────┐ │
│ │BURIAL ENVIRONMENT       │ │
│ │IDENTIFICATION UNIT      │ │
│ └─────────────────────────┘ │
│                     ⌐64e    │
│ ┌─────────────────────────┐ │
│ │PREPROCESSED             │ │
│ │BURIED PIPE DATA         │ │
│ │CREATION UNIT            │ │
│ └─────────────────────────┘ │
└─────────────────────────────┘
```

```
┌─────────────────────────────┐ ⌐66
│ PROBABILITY-OF-WATER-LEAKAGE-│
│ ACCIDENTS CALCULATION UNIT  │
└─────────────────────────────┘
```

```
┌─────────────────────────────┐ ⌐67
│ PROBABILITY-OF-WATER-LEAKAGE-│
│ ACCIDENTS PREDICTION RESULT │
│ OUTPUT UNIT                 │
└─────────────────────────────┘
```

## FIG.3

STORAGE UNIT — 20

BURIED PIPE DATA STORAGE UNIT — 21

BURIED PIPE DATA — 30

PIPELINE MAP — 31

BURIED PIPE ATTRIBUTE DATA — 32

NOMINAL PIPE WALL THICKNESS DATABASE UNIT — 22

MAP DATABASE UNIT — 23

BURIAL ENVIRONMENT CLASSIFICATION MAP STORAGE UNIT — 24

BURIAL ENVIRONMENT CLASSIFICATION PROVISIONAL MAP — 36

BURIAL ENVIRONMENT CLASSIFICATION MAP — 38

PREPROCESSED BURIED PIPE DATA STORAGE UNIT — 25

PROBABILITY-OF-WATER-LEAKAGE-ACCIDENTS STORAGE UNIT — 27

PROBABILITY-OF-WATER-LEAKAGE-ACCIDENTS PREDICTION MODEL STORAGE UNIT — 26

PROBABILITY-OF-WATER-LEAKAGE-ACCIDENTS PREDICTION MODEL — 42

⋮

PROBABILITY-OF-WATER-LEAKAGE-ACCIDENTS PREDICTION MODEL — 42

PROGRAM STORAGE UNIT — 28

BURIAL ENVIRONMENT CLASSIFICATION MAP CREATION PROGRAM — 47

PROBABILITY-OF-WATER-LEAKAGE-ACCIDENTS PREDICTION PROGRAM — 48

FIG.4

23

MAP DATABASE UNIT

34

FIRST GEOLOGICAL
INFORMATION MAP

34a

SUBSURFACE
GEOLOGICAL MAP
(LARGE CLASSIFICATION,
SMALL CLASSIFICATION)

34b

TOPOGRAPHY
CLASSIFICATION MAP
(LARGE CLASSIFICATION,
SMALL CLASSIFICATION)

35

SECOND GEOLOGICAL
INFORMATION MAP

35a

SUBSURFACE
GEOLOGICAL MAP
(SMALL CLASSIFICATION)

35b

COASTLINE MAP

35c

ELEVATION MAP

FIG.5

30(31)

FIG.6

30(32)

| PIPELINE ID | INSTALLATION YEAR | NOMINAL DIAMETER | TYPE OF JOINT | TYPE OF PIPE WALL THICKNESS | PIPELINE LENGTH (km) |
|---|---|---|---|---|---|
| ... | 1987 | 100 | K | 3 | ... |
| ... | 2000 | 150 | NS | 1 | ... |

FIG.7

33

| INSTALLATION YEAR | NOMINAL DIAMETER | TYPE OF JOINT | TYPE OF PIPE WALL THICKNESS | NOMINAL PIPE WALL THICKNESS (mm) |
|---|---|---|---|---|
| 1987 | 100 | K | 3 | ... |
| 2000 | 150 | NS | 1 | ... |

FIG.8

BURIAL
ENVIRONMENT

A
B
C
D

FIG.9

## FIG.10

| TOPOGRAPHY CLASSIFICATION MAP | | SUBSURFACE GEOLOGICAL MAP | | BURIAL ENVIRONMENT |
|---|---|---|---|---|
| LARGE CLASSIFICATION | SMALL CLASSIFICATION | LARGE CLASSIFICATION | SMALL CLASSIFICATION | |
| LOAM PLATEAU | VOLCANIC ASH PLATEAU (MIDDLE LEVEL) | CONSOLIDATED SEDIMENT | PEBBLE, ALTERNATING SHALE LAYER | A |
| LOW LAND | DRAINED LAND | UNCONSOLIDATED | SAND | |
| ⋮ | ⋮ | ⋮ | ⋮ | |
| LOW LAND | NATURAL EMBANKMENT, SANDBAR, SAND HILL | UNCONSOLIDATED | SAND (DUNE SAND) | B |
| PLATEAU TERRACE | GRAVEL PLATEAU (UPPER LEVEL) | UNCONSOLIDATED | GRAVEL, SAND, MUD | |
| ⋮ | ⋮ | ⋮ | ⋮ | |
| HILLY TERRAIN | SMALL UNDULATING HILLY TERRAIN | PLUTONIC ROCK | GRANITIC ROCK | C |
| LOW LAND | FAN-LIKE GEOLOGICAL LOW LAND | UNCONSOLIDATED | SAND | |
| ⋮ | ⋮ | ⋮ | ⋮ | |
| MOUNTAIN LAND | PIEDMONT | UNCONSOLIDATED | GRAVEL SEDIMENT | D |
| HILLY TERRAIN | VOLCANIC HILLY TERRAIN | VOLCANIC | TUFFACEOUS BRECCIA | |
| ⋮ | ⋮ | ⋮ | ⋮ | |

EP 4 567 403 A1

FIG.11

SPECIFIC
RESISTANCE OF
GROUNDWATER
(Ω cm)

MEDIAN

LARGE    MEDIUM    SMALL

DEGREE OF CORROSION
OF BURIED PIPE

FIG.12

SPECIFIC
RESISTANCE OF
GROUNDWATER
(Ω cm)

MEDIAN

~1    1~2    2~3    3~4    4~5    5~

DISTANCE FROM COASTLINE (km)

## FIG.13

| TOPOGRAPHY CLASSIFICATION MAP | |
|---|---|
| SMALL CLASSIFICATION | |
| SAND | GRAVEL-BASED SOIL |
| SAND (DUNE SAND) | |
| GRAVEL SEDIMENT | |
| ⋮ | |
| PEBBLE, ALTERNATING SHALE LAYER | NON-GRAVEL-BASED SOIL |
| GRAVEL, SAND, MUD | |
| GRANITIC ROCK | |
| TUFFACEOUS BRECCIA | |
| ⋮ | |

FIG.14

FIG.15

## FIG.16

| PIPELINE ID | BURIAL ENVIRONMENT | BURIAL PERIOD OF TIME (YEARS) | NOMINAL PIPE WALL THICKNESS (mm) |
|---|---|---|---|
| . . . | A | 44 | . . . |
| . . . | B | 21 | . . . |

40

## FIG.17

50(51)

| PROBABILITY OF WATER LEAKAGE ACCIDENTS | |
|---|---|
| ▬ | $\geq$ 0.5 CASE/YEAR/KM |
| ▬ | $\geq$ 0.1 CASE/YEAR/KM |
| — | $\geq$ 0.05 CASE/YEAR/KM |
| — | $<$ 0.05 CASE/YEAR/KM |

## FIG.18

50(52)

| PIPELINE ID | PROBABILITY OF WATER LEAKAGE ACCIDENTS (CASE/YEAR/KM) |
|---|---|
| . . . | . . . |
| . . . | . . . |

FIG.19

| | |
|---|---|
| CREATE BURIAL ENVIRONMENT CLASSIFICATION PROVISIONAL MAP 36 | S1 |
| CREATE BURIAL ENVIRONMENT CLASSIFICATION MAP 38 | S2 |

FIG.20

S2

| | |
|---|---|
| FIRST SOIL IDENTIFICATION STEP | S4 |
| SECOND SOIL IDENTIFICATION STEP | S5 |

FIG.21

| | |
|---|---|
| RECEIVE BURIED PIPE DATA 30 | S11 |
| CREATE PREPROCESSED BURIED PIPE DATA 40 | S12 |
| CALCULATE PROBABILITY OF WATER LEAKAGE ACCIDENTS FOR EACH PIPE ID | S13 |
| OUTPUT PROBABILITY-OF-WATER-LEAKAGE-ACCIDENTS PREDICTION RESULT 50 FOR EACH PIPE ID | S14 |

FIG.22

S12

S21

READ EMBEDDING PIPE DATA 30

S22

CALCULATE BURIAL PERIOD OF TIME OF BURIED PIPE

S23

IDENTIFY NOMINAL PIPE WALL THICKNESS OF BURIED PIPE

S24

IDENTIFY BURIAL ENVIRONMENT OF BURIED PIPE

S25

CREATE PREPROCESSED BURIED PIPE DATA 40

## INTERNATIONAL SEARCH REPORT

| International application No. |
| --- |
| **PCT/JP2023/027501** |

| A. | CLASSIFICATION OF SUBJECT MATTER |
| --- | --- |

*G01N 17/00*(2006.01)i; *G01N 33/24*(2006.01)i
FI:   G01N17/00; G01N33/24 B

According to International Patent Classification (IPC) or to both national classification and IPC

| B. | FIELDS SEARCHED |
| --- | --- |

Minimum documentation searched (classification system followed by classification symbols)

    G01N17/00-19/10; G01N33/00-33/46; E03B1/00-11/16; E03F1/00-11/00; F16L9/00-11/26

Documentation searched other than minimum documentation to the extent that such documents are included in the fields searched

    Published examined utility model applications of Japan 1922-1996
    Published unexamined utility model applications of Japan 1971-2023
    Registered utility model specifications of Japan 1996-2023
    Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | JP 2007-107882 A (KUBOTA CORP) 26 April 2007 (2007-04-26)<br>claims, paragraphs [0022]-[0026] | 1-6 |
| A | WO 2022/070706 A1 (KUBOTA CORP) 07 April 2022 (2022-04-07)<br>claims | 1-6 |

☐ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **06 September 2023** | **19 September 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/027501**

| Patent document cited in search report | | | Publication date (day/month/year) | Patent family member(s) | | | Publication date (day/month/year) |
|---|---|---|---|---|---|---|---|
| JP | 2007-107882 | A | 26 April 2007 | (Family: none) | | | |
| WO | 2022/070706 | A1 | 07 April 2022 | EP claims | 4224141 | A1 | |
| | | | | JP | 2021-56224 | A | |
| | | | | CA | 3194044 | A1 | |
| | | | | TW | 202221303 | A | |

Form PCT/ISA/210 (patent family annex) (January 2015)

## REFERENCES CITED IN THE DESCRIPTION

**Patent documents cited in the description**

- JP 2007107882 A

- JP 2021056224 A